(19) Europäisches Patentamt
European Patent Office
Office européen des brevets

(11) Veröffentlichungsnummer: **0 044 407**
**B1**

(12) # EUROPÄISCHE PATENTSCHRIFT

(45) Veröffentlichungstag der Patentschrift: **20.06.84**

(51) Int. Cl.³: **C 07 D 405/06,** C 07 D 405/14, C 07 D 409/14, A 01 N 43/64

(21) Anmeldenummer: **81104597.0**

(22) Anmeldetag: **15.06.81**

(54) **1,3-Dioxan-5-yl-alkyltriazole, ihre Herstellung, ihre Verwendung zur Regulierung des Pflanzenwachstums und Mittel dafür.**

(30) Priorität: **09.07.80 DE 3025879**

(43) Veröffentlichungstag der Anmeldung: **27.01.82 Patentblatt 82/4**

(45) Bekanntmachung des Hinweises auf die Patenterteilung: **20.06.84 Patentblatt 84/25**

(84) Benannte Vertragsstaaten: **AT BE CH DE FR GB IT LI LU NL SE**

(56) Entgegenhaltungen:
**DE - A - 2 739 352**
**FR - A - 2 290 898**
**FR - A - 2 434 164**
**FR - A - 2 440 367**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Feuerherd, Karl-Heinz, Dr., Berner Weg 34, D-6700 Ludwigshafen (DE)**
Erfinder: **Zeeh, Bernd, Dr., Thorwaldsenstrasse 5, D-6700 Ludwigshafen (DE)**
Erfinder: **Jung, Johann, Dr., Hardenburgstrasse 19, D-6703 Limburgerhof (DE)**

EP 0 044 407 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 1,3-Dioxan-5-ylalkyltriazole, Verfahren zu ihrer Herstellung, Mittel zur Regulierung des Pflanzenwachstums, die diese Verbindungen enthalten, und Verfahren zur Regulierung des Pflanzenwachstums mit diesen Verbindungen.

Es ist bekannt, dass bestimmte 2-Halogenethyltrialkylammoniumhalogenide pflanzenwachstumsregulierende Eigenschaften aufweisen (vgl. US-PS Nr. 3156554). So lässt sich mit Hilfe von (2-Chlorethyl)trimethylammoniumchlorid das Pflanzenwachstum beeinflussen. Allerdings ist die Wirksamkeit dieses Stoffes, vor allem bei niedrigen Aufwandmengen, nicht immer ausreichend.

Es ist ferner bekannt, 3,3-Dimethyl-2-[1,2,4-triazolyl-(1)]-1-(4-chlorbenzoyl)butan zur Regulierung des Pflanzenwachstums zu verwenden (DE-OS Nr. 2739352).

Es wurde nun gefunden, dass Verbindungen der Formel I

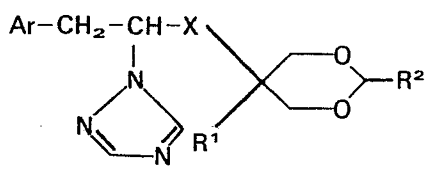
(I)

in der

$R^1$ und $R^2$ verschieden oder gleich sind und Wasserstoff oder Alkyl mit 1 bis 5 C-Atomen bedeuten,

Ar Furanyl, Thienyl, Biphenylyl, Naphthyl oder Phenyl bedeutet, wobei der Phenylrest durch Fluor, Chlor, Brom, Iod, Nitro, Trifluormethyl, weiterhin durch Alkyl, Alkoxy oder Alkenyl mit jeweils 1 bis 5 C-Atomen und ferner durch Phenoxy substituiert sein kann,

X eine $-CO-$, $-CH(OH)-$ oder $-CH(OR^3)-$-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder einen $-CO-R^4$- Rest bedeutet, wobei $R^4$ einen Alkylrest mit 1 bis 5 C-Atomen bedeutet, hervorragend zur Beeinflussung des Pflanzenwachstums geeignet sind und eine sehr gute Pflanzenverträglichkeit besitzen.

Die neuen Verbindungen der Formel I enthalten chirale Zentren und werden im allgemeinen in Form von Racematen oder als Diastereomerengemische von erythro- sowie threo-Formen erhalten. Die erythro- und threo-Diastereomeren lassen sich bei einigen der erfindungsgemässen Verbindungen beispielsweise auf Grund von Löslichkeitsunterschieden oder durch Säulenchromatographie trennen und in reiner Form isolieren. Aus solchen einheitlichen Diastereomerenpaaren kann man mit bekannten Methoden einheitliche Enantiomere erhalten. Diese werden von der vorliegenden Erfindung ebenfalls umfasst. Als Mittel zur Beeinflussung des Pflanzenwachstums kann man sowohl die einheitlichen Diastereomeren bzw. Enantiomeren wie auch deren bei der Synthese anfallenden Gemische verwenden. Bevorzugt werden die letzteren verwendet.

$R^1$ und $R^2$ bedeuten beispielsweise Wasserstoff, Methyl, Ethyl, n-Propyl, Isopropyl, n-Butyl, sek.-Butyl, Isobutyl, tert.-Butyl, n-Pentyl und Neopentyl.

Ar bedeutet beispielsweise 2-Furanyl, 2- und 3-Thienyl, 4-Biphenylyl, 1- und 2-Naphthyl, Phenyl, 4-Fluorphenyl, 2-Chlorphenyl, 3-Chlorphenyl, 4-Chlorphenyl, 4-Bromphenyl, 2,4-Dichlorphenyl, 3,4-Dichlorphenyl, 3,5-Dichlor-2-methoxyphenyl, 2,3,4-Trichlorphenyl, 2-Methoxyphenyl, 2,4-Dimethoxyphenyl, 3-Methoxyphenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 3-Nitrophenyl, 4-Nitrophenyl, 3-Trifluormethylphenyl, 4-Trifluormethylphenyl und 4-Phenoxyphenyl.

X bedeutet beispielsweise eine Carbonylgruppe ($C=O$) oder die daraus durch Reduktion resultierenden Alkohole ($-CHOH$), die man weiterhin verethern (zu $-CH-OR^3$) oder verestern (zu $-CH-OCOR^4$) kann. Dabei bedeutet beispielsweise $R^3$ Methyl, Ethyl, n-Propyl, n-Butyl, 4-Chlorbutyl, n-Pentyl, n-Hexyl, Allyl, Buten-2-yl, Propargyl, Benzyl, 4-Fluorbenzyl, 4-Chlorbenzyl, 4-Brombenzyl, 2,4-Dichlorbenzyl und 4-Trifluormethylbenzyl. $R^4$ bedeutet vorzugsweise Methyl, Ethyl, n-Propyl, Isopropyl, Chlormethyl, Chlorpropyl, Methoxymethyl.

Die neuen Verbindungen lassen sich herstellen, indem man

a) 1,2,4-Triazol mit α-Bromketonen der Formel II

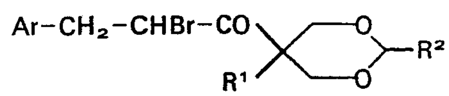
(II)

worin $R^1$, $R^2$ und Ar die oben angegebene Bedeutung haben, oder

b) ein Arylmethylhalogenid der Formel III

$$Ar-CH_2-Y \qquad (III)$$

worin Ar die oben genannte Bedeutung hat und Y Chlor oder Brom ist, mit einem 1-(1,3-Dioxan-5-yl)-2-[1,2,4-triazolyl-(s)]-ethan-1-on der Formel IV

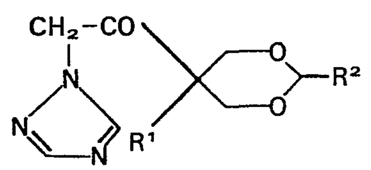
(IV)

worin $R^1$ und $R^2$ die oben genannte Bedeutung haben,

umsetzt und die so erhaltenen Verbindungen gegebenenfalls anschliessend reduziert und – falls gewünscht – danach verethert oder verestert.

Die Reaktion a erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120° C. Zu den bevorzugten Lösungs- und Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid; alkalicarbonate wie Natrium- oder Kaliumcarbonat oder Natrium- und Kaliumhydrogencarbonat, Überschüsse an 1,2,4-Triazol, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Bentyltriethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzungen werden im allgemeinen bei Temperaturen zwischen 20 und 150°C, drucklos oder unter Druck, kontinuierlich oder diskontinuierlich durchgeführt.

Die Bromketone der Formel II sind neu. Sie können beispielsweise durch Bromierung von Verbindungen der Formel V

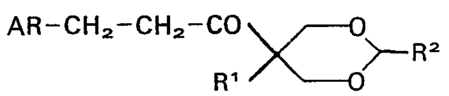 (V)

in welcher Ar, $R^1$ und $R^2$ die oben angegebenen Bedeutungen haben mit Brom in Formamid nach H. Bredereck *et al.*, „Chem. Ber.", *93*, 2083 (1960) oder mit Dioxandibromid nach S.J. Pasaribu und L.R. Williams, „Aust. J. Chem.", *26*, 1327 (1973) oder mit dem Komplex (Pyrrolidon)$_3$.HBr·$Br_2$ nach D.C. Awang *et al.*, „Can. J. Chem.", *47*, 706 (1969), erhalten werden.

Die Reaktion b läuft gegebenenfalls in Gegenwart eines Lösungs- oder Verdünnungsmittels und gegebenenfalls unter Zusatz einer starken anorganischen oder organischen Base bei Temperaturen zwischen −10 und 120°C ab. Zu den bevorzugten Lösungs- oder Verdünnungsmitteln gehören Amide wie Dimethylformamid, Diethylformamid, Dimethylacetamid, Diethylacetamid, N-Methylpyrrolidon, Hexamethylphosphortriamid, Sulfoxide wie Dimethylsulfoxid und schliesslich Sulfolan.

Geeignete Basen, die gegebenenfalls auch als säurebindende Mittel bei der Reaktion verwendet werden können, sind beispielsweise Alkalihydride, wie Lithium-, Natrium- und Kaliumhydrid, Alkaliamide wie Natrium- und Kaliumamid, ferner Natrium- oder Kalium.-tert.-butoxid, Lithium-, Natrium- oder Kaliumtriphenylmethyl und Naphthalinlithium, -natrium oder -kalium.

Die 1-(1,3-Dioxan-5-yl)-2-[1,2,4-triazolyl-(1)]ethan-1-one der Formel IV sind neu. Sie können beispielsweise durch Umsetzung von 2-Halogen-1-(2,5-dialkyl-1,3-dioxan-5-yl)ethan-1-onen der Formel VI

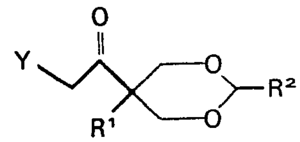 (VI)

in welcher $R^1$, $R^2$ und Y die oben angegebenen Bedeutungen haben, mit 1,2,4-Triazol oder dessen Alkalisalz in einem geeigneten Lösungsmittel erhalten werden.

Halogenketone der Formel VI sind auch neu. Man erhält sie durch z.B. Bromierung von bekannten (1,3-Dioxan-5-yl)ethan-1-onen der Formel VII

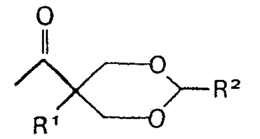 (VII)

in welcher $R^1$ und $R^2$ die oben angegebenen Bedeutungen haben, beispielsweise mit dem Pyrrolidon/Brom-Komplex nach D.P. Wyman und P.R. Kaufman, „J. Org. Chem.", *29*, 1956 (1964).

Die gemäss a oder b erhaltenen Ketone lassen sich gegebenenfalls in Gegenwart eines Lösungs- oder Verdünnungsmittels, bei Temperaturen zwischen −20 bis 150°C, bei Normaldruck oder unter erhöhtem Druck mit Wasserstoff in Gegenwart eines Katalysators, mit komplexen Bor- bzw. Aluminiumhydriden, mit Aluminiumisopropoxid und Isopropanol, mit Natriumdithionit oder elektrochemisch reduzieren.

Geeignete Lösungs- oder Verdünnungsmittel hierfür sind beispielsweise Wasser, Methanol, Ethanol, Isopropanol, Essigsäure, Diethylether, Tetrahydrofuran, Dioxan, Essigester, Toluol, Dimethylformamid oder entsprechende Gemische.

Zur katalytischen Hydrierung verwendet man Platin- oder Palladiumkatalysatoren auf inerten Trägern und hydriert bei Drucken von 2 bis 80 bar bis keine Wasserstoffaufnahme mehr erfolgt.

Für die als Reduktionsmittel verwendbaren Hydride seien beispielsweise Natriumborhydrid oder Lithiumaluminiumhydrid genannt.

Die so erhaltenen Alkohole der Formel I (X=CHOH) lassen sich mit Alkylierungsmitteln der Formel VII

$$Z-R^3$$

worin $R^3$ die oben angegebene Bedeutung hat und Z Chlor oder Brom darstellt, gegebenenfalls in Gegenwart eines Lösungs- oder Verdünnungsmittels die Ein- oder Zweiphasensysteme bilden, gegebenenfalls unter Zusatz einer anorganischen Base und gegebenenfalls unter Zusatz eines Reaktionsbeschleunigers und/oder Phasentransferkatalysators verethern.

Hierfür eignen sich folgende Lösungs- oder Verdünnungsmittel: Diethylether, Tetrahydrofuran, Dioxan, n-Pentan, monohalogenierte Kohlenwasserstoffe mit 2 bis 6 C-Atomen wie beispielsweise Chlorethan, Bromethan, 1-Chlorpropan, 1-Brompropan, 1-Chlorbutan, 1-Chlorpentan, 1-Brompentan, 1-Chlorhexan, 1-Bromhexan, ferner Cyclohexan, Methylenchlorid, Chloroform, Toluol oder Dimethylformamid.

Als anorganische Basen seien beispielsweise genannt: Alkali- und Erdalkalihydroxide wie Natriumhydroxid, Kaliumhydroxid, Calciumhydroxid, Bariumhydroxid, Alkali- und Erdalkalicarbonate wie Natriumcarbonat, Kaliumcarbonat, Natriumhydrogencarbonat, Kaliumhydrogencarbonat, Calciumcarbonat, Bariumcarbonat, Alkali- oder Erdalkalialkoholate wie Natriummethylat, Natriumethylat, Magnesiummethylat, Natriumisopropylat oder Kalium-tert.-butylat.

Geeignete Reaktionsbeschleuniger sind beispielsweise Metallhalogenide wie Natriumbromid, Natriumiodid, Kaliumbromid, Kaliumiodid, tertiäre Amine wie 4-Dimethylaminopyridin und 4-Pyrrolidinopyridin, Kronenether wie 12-Krone-4, 14-Krone-5, 18-Krone-6, Dibenzo-18-Krone-6 oder Dicyclohexano-18-Krone-6 oder Azole wie Imidazol und 1,2,4-Triazol.

Als Phasentransferkatalysatoren kommen vorzugsweise quaternäre Ammoniumsalze wie Tetrabutylammoniumchlorid, -bromid, -iodid oder -hydrogensulfat, Benzyltriethylammoniumchlorid, Methyltrioctylammoniumchlorid und -bromid und Phosphoniumsalze wie Tetrabutylphophoniumbromid und -iodid und Tetra-n-pentylphosphoniumbromid und -iodid in Frage.

Die Veresterung der Alkohole der Formel I (X=CHOH) kann mit Säurechloriden oder -anhydriden der Formel VIII ($Y-CO-R^4$) oder der Formel IX [$(R^4-CO)_2O$] gegebenenfalls in Gegenwart eines Lösungs- oder Verdünnungsmittels, gegebenenfalls unter Zusatz eines säurebindenden Mittels und gegebenenfalls unter Zusatz eines Reaktionsbeschleunigers erfolgen. Hierfür eignen sich dieselben Lösungsmittel und Basen wie für die Veretherung. Zusätzlich können tertiäre Amine wie Trimethylamin, Triethylamin, N,N-Dimethylanilin, N,N-Dimethylcyclohexylamin, N-Methylpiperidin oder Pyridin als Base verwendet werden.

Mit geeigneten Basen wie z.B. Alkalihydrid wie Natriumhydrid oder Alkali- oder Erdalkalialkoholaten wie Natriummethylat können die Alkohole auch in einer ersten Umsetzung zunächst in ihre Alkoholatsalze überführt werden und dann als solche zur Reaktion gebracht werden.

Die so erhaltenen Verbindungen der Formel I werden nach üblichen Methoden isoliert, gegebenenfalls gereinigt und gegebenenfalls mit Säuren oder Metallsalzen zu Salzen bzw. zu Metallkomplexen umgesetzt.

Als Beispiele für die neuen erfindungsgemässen Verbindungen der Formel I seien im einzelnen genannt:

1-(5-Methyl-1,3-dioxan-5-yl)-2-[1,2,4-triazolyl-(1)]-3-phenylpropan-1-on,
1-(5-Methyl-1,3-dioxan-5-yl)-2-[1,2,4-triazolyl-(1)]-3-phenylpropan-1-ol,
1-(5-Methyl-1,3-dioxan-5-yl)-1-methoxy-2-[1,2,4-triazolyl-(1)]-3-phenylpropan,
1-(5-Methyl-1,3-dioxan-5-yl)-1-ethoxy-2-[1,2,4-triazolyl-(1)]-3-phenylpropan,
1-(5-Methyl-1,3-dioxan-5-yl)-1-propoxy-2-[1,2,4-triazolyl-(1)]-3-phenylpropan,
1-(5-Methyl-1,3-dioxan-5-yl)-1-n-butoxy-2-[1,2,4-triazolyl-(1)]-3-phenylpropan,
1-(5-Methyl-1,3-dioxan-5-yl)-1-n-pentyloxy-2-[1,2,4-triazolyl-(1)]-3-phenylpropan,
1-(5-Methyl-1,3-dioxan-5-yl)-1-allyloxy-2-[1,2,4-triazolyl-(1)]-3-phenylpropan,
1-(5-Methyl-1,3-dioxan-5-yl)-1-propargyloxy-2-[1,2,4-triazolyl-(1)]-3-phenylpropan,
1-(5-Methyl-1,3-dioxan-5-yl)-1-acetoxy-2-[1,2,4-triazolyl-(1)]-3-phenylpropan,
1-(5-Methyl-1,3-dioxan-5-yl)-1-propionyloxy-2-[1,2,4-triazolyl-(1)]-3-phenylpropan,
1-(2-Ethyl-5-methyl-1,3-dioxan-5-yl)-2-[1,2,4-triazolyl-(1)]-3-phenylpropan-1-on,
1-(2-Ethyl-5-methyl-1,3-dioxan-5-yl)-2-[1,2,4-triazolyl-(1)]-3-phenylpropan-1-ol,
1-(5-Methyl-1,3-dioxan-5-yl)-2-[1,2,4-triazolyl-(1)]-3-(1-naphthyl)propan-1-on,
1-(5-Methyl-1,3-dioxan-5-yl)-2-[1,2,4-triazolyl-(1)]-3-(1-naphthyl)propan-1-ol,
1-(5-Methyl-1,3-dioxan-5-yl)-1-methoxy-2-[1,2,4-triazolyl-(1)]-3-(1-naphthyl)propan,
1-(5-Methyl-1,3-dioxan-5-yl)-1-n-propoxy-2-[1,2,4-triazolyl-(1)]-3-(1-naphthyl)propan,
1-(5-Methyl-1,3-dioxan-5-yl)-1-n-butoxy-2-[1,2,4-triazolyl-(1)]-3-(1-naphthyl)propan,
1-(5-Methyl-1,3-dioxan-5-yl)-1-allyloxy-2-[1,2,4-triazolyl-(1)]-3-(1-naphthyl)propan,
1-(5-Methyl-1,3-dioxan-5-yl)-1-acetoxy-2-[1,2,4-triazolyl-(1)]-3-(1-naphthyl)propan,
1-(5-Methyl-1,3-dioxan-5-yl)-1-(chloracetoxy)-2-[1,2,4-triazolyl-(1)]-3-(1-naphthyl)propan,
1-(5-Methyl-1,3-dioxan-5-yl)-1-propionyloxy-2-[1,2,4-triazolyl-(1)]-3-(1-naphthyl)propan,
1-(2-Ethyl-5-methyl-1,3-dioxan-5-yl)-2-[1,2,4-triazolyl-(1)]-3-(1-naphthyl)propan-1-on,
1-(2-Ethyl-5-methyl-1,3-dioxan-5-yl)-2-[1,2,4-triazolyl-(1)]-3-(1-naphthyl)propan-1-ol,
1-(2-Propyl-5-methyl-1,3-dioxan-5-yl)-2-[1,2,4-triazolyl-(1)]-3-(1-naphthyl)propan-1-on,
1-(2-Ethyl-5-methyl-1,3-dioxan-5-yl)-1-acetoxy-2-[1,2,4-triazolyl-(1)]-3-(1-naphthyl)propan,
1-(5-Methyl-1,3-dioxan-5-yl)-2-[1,2,4-triazolyl-(1)]-3-(4-fluorphenyl)propan-1-on,
1-(5-Methyl-1,3-dioxan-5-yl)-2-[1,2,4-triazolyl-(1)]-3-(4-fluorphenyl)propan-1-ol,
1-(5-Methyl-1,3-dioxan-5-yl)-1-n-propoxy-2-[1,2,4-triazolyl-(1)]-3-(4-fluorphenyl)propan,
1-(5-Methyl-1,3-dioxan-5-yl)-1-acetoxy-2-[1,2,4-triazolyl-(1)]-3-(4-fluorphenyl)propan,
1-(2-Ethyl-5-methyl-1,3-dioxan-5-yl)-2-[1,2,4-triazolyl-(1)]-3-(4-fluorphenyl)propan-1-on,
1-(2-Ethyl-5-methyl-1,3-dioxan-5-yl)-2-[1,2,4-triazolyl-(1)]-3-(4-fluorphenyl)propan-1-ol,
1-(5-Methyl-1,3-dioxan-5-yl)-2-[1,2,4-triazolyl-(1)]-3-(4-chlorphenyl)propan-1-on,
1-(5-Methyl-1,3-dioxan-5-yl)-2-[1,2,4-triazolyl-(1)]-3-(4-chlorphenyl)propan-1-ol,
1-(2,5-Dimethyl-1,3-dioxan-5-yl)-2-[1,2,4-triazolyl-(1)]-3-(4-chlorphenyl)propan-1-on,
1-(2,5-Dimethyl-1,3-dioxan-5-yl)-2-[1,2,4-triazolyl-(1)]-3-(4-chlorphenyl)propan-1-ol,
1-(2-Ethyl-5-methyl-1,3-dioxan-5-yl)-2-[1,2,4-triazolyl-(1)]-3-(4-chlorphenyl)propan-1-on,
1-(2-Ethyl-5-methyl-1,3-dioxan-5-yl)-2-[1,2,4-triazolyl-(1)]-3-(4-chlorphenyl)propan-1-ol,
1-(2-n-Propyl-5-methyl-1,3-dioxan-5-yl)-2-[1,2,4-triazolyl-(1)]-3-(4-chlorphenyl)propan-1-on,
1-(2-n-Propyl-5-methyl-1,3-dioxan-5-yl)-2-[1,2,4-triazolyl-(1)]-3-(4-chlorphenyl)propan-1-ol,
1-(2-Isopropyl-5-methyl-1,3-dioxan-5-yl)-2-[1,2,4-triazolyl-(1)]-3-(4-chlorphenyl)propan-1-on,
1-(2-Isopropyl-5-methyl-1,3-dioxan-5-yl)-2-[1,2,4-triazolyl-(1)]-3-(4-chlorphenyl)propan-1-ol,
1-(2-n-Butyl-5-methyl-1,3-dioxan-5-yl)-2-[1,2,4-triazolyl-(1)]-3-(4-chlorphenyl)propan-1-on,
1-(2-n-Butyl-5-methyl-1,3-dioxan-5-yl)-2-[1,2,4-triazolyl-(1)]-3-(4-chlorphenyl)propan-1-ol,
1-(5-Methyl-1,3-dioxan-5-yl)-1-methoxy-2-[1,2,4-triazolyl-(1)]-3-(4-chlorphenyl)propan,
1-(5-Methyl-1,3-dioxan-5-yl)-1-n-propoxy-2-[1,2,4-triazolyl-(1)]-3-(4-chlorphenyl)propan,
1-(5Methyl-1,3-dioxan-5-yl)-1-n-butoxy-2-[1,2,4-triazolyl-(1)]-3-(4-chlorphenyl)propan,
1-(5-Methyl-1,3-dioxan-5-yl)-1-acetoxy-2-[1,2,4-triazolyl-(1)]-3-(4-chlorphenyl)propan,
1-(2-Ethyl-5-methyl-1,3-dioxan-5-yl)-1-methoxy-2-[1,2,4-triazolyl-(1)]-3-(4-chlorphenyl)propan,
1-(2-Ethyl-5-methyl-1,3-dioxan-5-yl)-1-n-propoxy-2-[1,2,4-triazolyl-(1)]-3-(4-chlorphenyl)propan,
1-(2-Ethyl-5-methyl-1,3-dioxan-5-yl)-1-acetoxy-2-[1,2,4-triazolyl-(1)]-3-(4-chlorphenyl)-propan,
1-(2-Ethyl-5-methyl-1,3-dioxan-5-yl)-1-propionyloxy-2-[1,2,4-triazolyl-(1)]-3-(4-chlorphenyl)propan,
1-(2-Ethyl-5-methyl-1,3-dioxan-5-yl)-1-butyryloxy-2-[1,2,4-triazolyl-(1)]-3-(4-chlorphenyl)propan,
1-(5-Methyl-1,3-dioxan-5-yl)-2-[1,2,4-triazolyl-(1)]-3-(4-bromphenyl)propan-1-on,
1-(5-Methyl-1,3-dioxan-5-yl)-2-[1,2,4-triazolyl-(1)]-3-(4-bromphenyl)propan-1-ol,
1-(2-Ethyl-5-methyl-1,3-dioxan-5-yl)-2-[1,2,4-triazolyl-(1)]-3-(4-bromphenyl)propan-1-on,
1-(2-Ethyl-5-methyl-1,3-dioxan-5-yl)-2-[1,2,4-triazolyl-(1)]-3-(4-bromphenyl)propan-1-ol,
1-(5-Methyl-1,3-dioxan-5-yl)-1-acetoxy-2-[1,2,4-triazolyl-(1)]-3-(4-bromphenyl)propan,
1-(2-Ethyl-5-methyl-1,3-dioxan-5-yl)-1-acetoxy-2-[1,2,4-triazolyl-(1)]-3-(4-bromphenyl)-propan,
1-(5-Methyl-1,3-dioxan-5-yl)-2-[1,2,4-triazolyl-(1)]-3-(2,4-dichlorphenyl)propan-1-on,
1-(5-Methyl-1,3-dioxan-5-yl)-2-[1,2,4-triazolyl-(1)]-3-(2,4-dichlorphenyl)propan-1-ol,
1-(2-Ethyl-5-methyl-1,3-dioxan-5-yl)-2-[1,2,4-triazolyl-(1)]-3-(2,4-dichlorphenyl)propan-1-on,
1-(2-Ethyl-5-methyl-1,3-dioxan-5-yl)-2-[1,2,4-triazolyl-(1)]-3-(2,4-dichlorphenyl)propan-1-ol,
1-(2,5-Methyl-1,3-dioxan-5-yl)-2-[1,2,4-triazolyl-(1)]-3-(2,4-dichlorphenyl)propan-1-on,
1-(2,5-Diethyl-1,3-dioxan-5-yl)-2-[1,2,4-triazolyl-(1)]-3-(2,4-dichlorphenyl)propan-1-ol,
1-(2-n-Propyl-5-methyl-1,3-dioxan-5-yl)-2-[1,2,4-triazolyl-(1)]-3-(2,4-dichlorphenyl)propan-1-ol,
1-(2-n-Propyl-5-methyl-1,3-dioxan-5-yl)-2-[1,2,4-triazolyl-(1)]-3-(2,4-dichlorphenyl)propan-1-on,
1-(2-Isopropyl-5-methyl-1,3-dioxan-5-yl)-2-[1,2,4-triazolyl-(1)]-3-(2,4-dichlorphenyl)propan-1-on,
1-(2-Isopropyl-5-methyl-1,3-dioxan-5-yl)-2-[1,2,4-triazolyl-(1)]-3-(2,4-dichlorphenyl)propan-1-ol,
1-(2-n-Butyl-5-methyl-1,3-dioxan-5-yl)-2-[1,2,4-triazolyl-(1)]-3-(2,4-dichlorphenyl)propan-1-on,
1-(2-n-Butyl-5-methyl-1,3-dioxan-5-yl)-2-[1,2,4-triazolyl-(1)]-3-(2,4-dichlorphenyl)propan-1-ol,
1-(2-tert.-Butyl-5-methyl-1,3-dioxan-5-yl)-2-[1,2,4-triazolyl-(1)]-3-(2,4-dichlorphenyl)propan-1-on,
1-(2-tert.-Butyl-5-methyl-1,3-dioxan-5-yl)-2-[1,2,4-triazolyl-(1)]-3-(2,4-dichlorphenyl)propan-1-ol,
1-(5-Methyl-1,3-dioxan-5-yl)-1-acetoxy-2-[1,2,4-triazolyl-(1)]-3-(2,4-dichlorphenyl)propan,
1-(2-Ethyl-5-methyl-1,3-dioxan-5-yl)-1-acetoxy-2-[1,2,4-triazolyl-(1)]-3-(2,4-dichlorphenyl)propan,
1-(2-Isopropyl-5-methyl-1,3-dioxan-5-yl)-1-acetoxy-2-[1,2,4-triazolyl-(1)]-3-(2,4-dichlorphenyl)propan,
1-(5-Methyl-1,3-dioxan-5-yl)-2-[1,2,4-triazolyl-(1)]-3-(3,4-dichlorphenyl)propan-1-on,
1-(5-Methyl-1,3-dioxan-5-yl)-2-(1,2,4-triazolyl-(1))-3-(3,4-dichlorphenyl)-propan-1-ol,
1-(2-Ethyl-5-methyl-1,3-dioxan-5-yl)-2-(1,2,4-triazolyl-(1))-3-(3,4-dichlorphenyl)propan-1-ol,
1-(2-Ethyl-5-methyl-1,2-dioxan-5-yl)-2-[1,2,4-triazolyl-(1)]-3-(3,4-dichlorphenyl)propan-1-on,
1-(2-Ethyl-5-methyl-1,2-dioxan-5-yl)-1-acetacetoxy-2-[1,2,4-triazolyl-(1)]-3-(3,4-dichlorphenyl)propan,
1-(5-Methyl-1,2-dioxan-5-yl)-2-[1,2,4-triazolyl-(1)]-3-(4-methylphenyl)propan-1-on,
1-(5-Methyl-1,3-dioxan-5-yl)-2-[1,2,4-triazolyl-(1)]-3-(4-methylphenyl)propan-1-ol,
1-(2-Ethyl-5-methyl-1,3-dioxan-5-yl)-2-[1,2,4-triazolyl-(1)]-3-(4-methylphenyl)propan-1-on,
1-(2-Ethyl-5-methyl-1,3-dioxan-5-yl)-2-[1,2,4-triazolyl-(1)]-3-(4-methylphenyl)propan-1-ol,
1-(5-Methyl-1,3-dioxan-5-yl)-2-[1,2,4-triazolyl-(1)]-3-(3-methylphenyl)propan-1-on,
1-(5-Methyl-1,3-dioxan-5-yl)-2-[1,2,4-triazolyl-(1)]-3-(3-methylphenyl)propan-1-ol,
1-(5-Methyl-1,3-dioxan-5-yl)-2-[1,2,4-triazolyl-(1)]-3-(4-ethylphenyl)propan-1-on,
1-(5-Methyl-1,3-dioxan-5-yl)-2-[1,2,4-triazolyl-(1)]-3-(4-ethylphenyl)propan-1-ol,
1-(5-Methyl-1,3-dioxan-5-yl)-2-[1,2,4-triazolyl-(1)]-3-(3,4-dimethylphenyl)propan-1-on,
1-(5-Methyl-1,3-dioxan-5-yl)-2-[1,2,4-triazolyl-(1)]-3-(3,4-dimethylphenyl)propan-1-ol,
1-(2-Ethyl-5-methyl-1,3-dioxan-5-yl)-2-[1,2,4-triazolyl-(1)]-3-(4-methoxyphenyl)propan-1-on,
1-(2-Ethyl-5-methyl-1,3-dioxan-5-yl)-2-[1,2,4-triazolyl-(1)]-3-(4-methoxyphenyl)propan-1-ol,
1-(2-Ethyl-5-methyl-1,3-dioxan-5-yl)-2-[1,2,4-triazolyl-(1)]-3-(4-nitrophenyl)propan-1-on,
1-(5-Methyl-1,3-dioxan-5-yl)-2-[1,2,4-triazolyl-(1)]-3-(4-trifluormethylphenyl)propan-1-on,
1-(5-Methyl-1,3-dioxan-5-yl)-2-[1,2,4-triazolyl-(1)]-3β-(4-trifluormethylphenyl)propan-1-ol,
1-(2-Ethyl-5-methyl-1,3-dioxan-5-yl)-2-[1,2,4-triazolyl-(1)]-3-(4-trifluormethylphenyl)propan-1-on,
1-(2-Ethyl-5-methyl-1,3-dioxan-5-yl)-2-[1,2,4-triazolyl-(1)]-3-(4-trifluormethylphenyl)propan-1-ol,
1-(2-Ethyl-5-methyl-1,3-dioxan-5-yl)-2-[1,2,4-triazolyl-(1)]-3-(4-tert.-Butylphenyl)propan-1-on,
1-(5-Methyl-1,3-dioxan-5-yl)-1-[1,2,4-triazolyl-(1)]-3-(4-ethoxyphenyl)propan-1-on,
1-(5-Methyl-1,3-dioxan-5-yl)-2-[1,2,4-triazolyl-(1)]-3-(4-ethoxyphenyl)propan-1-on.

Die folgenden Beispiele erläutern die Herstellung der Substanzen:

*Beispiel 1:*

a) Herstellung des Ausgangsmaterials

Zu einer Lösung von 144 g (1 mol) 5-Acetyl-5-methyl-1,3-dioxan und 85,5 g (1 mol) Pyrrolidon in 500 ml Tetrahydrofuran wird in 2 h bei 50°C die Lösung von 498 g (1 mol) Pyrrolidon/Brom-Komplex in 1 l Tetrahydrofuran zugetropft. Nach 8stündigem Nachrühren bei 50°C wird der weisse Niederschlag von Pyrrolidon/Hydrobromid abgesaugt, mit 50 ml Tetrahydrofuran gewaschen und das Filtrat im Vakuum eingeengt. Man erhält 220 g (99%) rohes, öliges 1-(5-Methyl-1,3-dioxan-5-yl)-2-bromethan-1-on.

Zu einer unter reinem Stickstoff gerührten Suspension von 100,1 g (1,1 mol) Natrium-1,2,4-triazolid in 300 ml trockenem Tetrahydrofuran wird die Lösung von 223 g (1 mol) 1-(5-Methyl-1,3-dioxan-5-yl)-2-bromethan-1-on in 200 ml Tetrahydrofuran bei 25°C in 2 h zugetropft. Nach 8stündigem Erhitzen unter Rückfluss wird der anorganische Niederschlag abfiltriert und das Filtrat zur Hälfte eingeengt. Das Gemisch wird angeimpft und über Nacht bei +3°C stehen gelassen. Der Niederschlag wird abgesaugt, mit 30 ml kaltem (+5°C) Tetrahydrofuran, dann mit 80 ml Ether und anschliessend mit 100 ml n-Pentan gewaschen und getrocknet. Man erhält 184 g (87,2%) 1-(5-Methyl-1,3-dioxan-5-yl)-2-[1,2,4-triazolyl-(1)]-ethan-1-on als weisse Kristalle vom Schmelzpunkt 95 bis 97°C.

b) Herstellung des Endproduktes

Zu einer unter reinem Stickstoff gerührten Suspension von 13,2 g (0,55 mol) Natriumhydrid in 100 ml trockenem Dimethylformamid wird die Lösung von 105,5 g (0,5 mol) 1-(5-Methyl-1,3-dioxan-5-yl)-2-[1,2,4-triazolyl-(1)]-ethan-1-on in 100 ml Dimethylformamid bei 20 bis 25°C zugetropft. Nach 3stündigem Nachrühren wird bei 25°C die Lösung von 81 g (0,5 mol) 4-Chlorbenzylchlorid in 50 ml Dimethylformamid zugetropft und das Reaktionsgemisch weitere 14 h nachgerührt. 50 ml Eiswasser werden vorsichtig zugetropft und das Gemisch wird im Vakuum eingeengt. Der Rückstand wird zwischen 400 ml Methylenchlorid und 200 ml Wasser verteilt, die organische Phase dreimal mit je 200 ml Wasser gewaschen, über $Na_2SO_2$ getrocknet und eingeengt. Man erhält 112 g (66,8%) 1-(5-Methyl-1,3-dioxan-5-yl)-2-[1,2,4-triazolyl-(1)]-3-(4-chlorphenyl)propan-1-on als blassgelbes Harz.

$^1H$-NMR (80 MHz/$CDCl_3$): δ=0,87 (s, 3H), 3,1-3,6 (m, 4H), 3,95-4,4 (t, 2H), 4,5-5,0 (2 dd, zus. 2H), 5,65-5,95 (q, 1H), 6,8-7,3 (m, 4H), 7,8 (s, 1H), 8,0 ppm (s, 1H).

*Beispiel 2:*

Zu einer Lösung von 90 g (0,269 mol) 1-(5-Methyl-1,3-dioxan-5-yl)-2-[1,2,4-triazolyl-(1)]-3-(4-chlorphenyl)propan-1-on in 250 ml Methanol werden zwischen 0 und +5°C 11,5 g (0,3 mol) Natriumhydrid portionsweise zugegeben. Nach 12stündigem Rühren bei 20°C wird das Gemisch eingeengt. Der Rückstand wird mit 200 ml 20%iger Kalilauge 1 h gerührt und mit 500 ml Methylenchlorid extrahiert. Die organische Phase wird dreimal mit je 50 ml Wasser gewaschen, über Natriumsulfat getrocknet und eingeengt. Der Rückstand kristallisiert bei +5°C nach Zugabe von 20 ml Ether. Man isoliert 45 g (49,6%) 1-(5-Methyl-1,3-dioxan-5-yl)-2-[1,2,4-triazolyl-(1)]-3-(4-chlorphenyl)propan-1-ol als weisse Kristalle vom Schmelzpunkt 152 bis 154°C.

*Beispiel 3:*

Unter kräftigem Rühren wird eine Mischung aus 15,2 g (0,045 mol) 1-(5-Methyl-1,3-dioxan-5-yl)-2-[1,2,4-triazolyl-(1)]-3-(4-chlorphenyl)-propan-1-ol, 100 g 1-Chlorpropan, 3 g Tetrabutylammoniumhydrogensulfat und 65 g 50%iger Natronlauge 36 h auf 30°C erwärmt. Die Mischung wird sodann mit 300 ml Wasser versetzt und zweimal mit je 150 ml Methylenchlorid extrahiert. Die vereinigten Extrakte werden achtmal mit je 100 ml Wasser ausgeschüttelt, über Magnesiumsulfat getrocknet und eingeengt. Der Rückstand kristallisiert nach Zugabe von 20 ml Petrolether und 5 ml Ether. Man isoliert 12 g (70,3%) 1-(5-Methyl-1,3-dioxan-5-yl)-1-n-propoxy-2-[1,2,4-triazolyl-(1)]-3-(4-chlorphenyl)propan als weisse Kristalle vom Schmelzpunkt 94 bis 96°C.

*Beispiel 4:*

Zu einer Suspension von 2,4 g Natriumhydrid in 120 ml trockenem Tetrahydrofuran wird die Lösung von 23,6 g (0,07 mol) 1-(5-Methyl-1,3-dioxan-5-yl)-2-[1,2,4-triazolyl-(1)]-3-(4-chlorphenyl)propan-1-ol in 100 ml Tetrahydrofuran getropft. Nach 12stündigem Rühren bei 25°C wird das Gemisch mit der Lösung von 9,7 g (0,08 mol) Allylbromid in 20 ml Tetrahydrofuran zugetropft. Nach 36stündigem Rühren wird das Reaktionsgemisch vorsichtig mit 20 ml Wasser versetzt und eingeengt. Der Rückstand wird in 350 ml Methylenchlorid aufgenommen, dreimal mit je 100 ml Wasser gewaschen, die organische Phase getrocknet und eingeengt. Der Rückstand wird mit 30 ml Petrolether und 10 ml Ether über Nacht bei +3°C gelassen. Der kristalline, farblose Niederschlag wird abgesaugt, mit Petrolether gewaschen und getrocknet. Man erhält 22,6 g (87,5%) 1-(5-Methyl-1,3-dioxan-5-yl)-1-allyloxy-2-[1,2,4-triazolyl-(1)]-3-(4-chlorphenyl)propan vom Schmelzpunkt 113 bis 115°C.

*Beispiel 5:*

Eine Mischung aus 20 g (0,053 mol) 1-(5-Methyl-1,3-dioxan-5-yl)-2-[1,2,4-triazolyl-(1)]-3-(2,4-dichlorphenyl)propan-1-ol (vgl. Beispiel 35), 2 g Imidazol und 100 ml Propionanhydrid werden 10 h bei 60°C gerührt und anschliessend im Vakuum eingeengt. Der Rückstand wird in 250 ml Ether gelöst und 30 min mit 100 ml einer 6%igen Natriumhydrogencarbonatlösung gerührt. Die organische Phase wird über Natriumsulfat getrocknet und im Vakuum – schliesslich bis 50°C und 0,1 mbar – eingeengt. Man erhält 17,1 g (75,4%) 1-(5-Methyl-1,3-dioxan-5-yl)-1-acetoxy-[1,2,4-triazolyl-(1)]-3-(2,4-dichlorphenyl)-propan als hellbraunes Harz.

$^1$H-NMR (80 MHz/$CDCl_3$): δ=0,85 (s, 3H), 1,15-1,4 (t, 3H), 2,4-3,2 (q, 2H), 3,3-3,6 (m, 2H), 3,8-4,2 (m, 2H), 4,6-5,1 (2 dd, zus. 2H), 6,1 (s, 1H), 6,5-7,6 (2 tt, zus. 4H), 8,1 (s, 1H) und 8,28 ppm (s, 1H).

In entsprechender Weise können die in der folgenden Tabelle aufgeführten Verbindungen hergestellt werden.

| Beispiel Nr. | Ar | R$^1$ | R$^2$ | X | Fp/°C IR (Film) [cm$^{-1}$] |
|---|---|---|---|---|---|
| 6 | $C_6H_5-$ | $CH_3$ | H | -CO- | 96-98 |
| 7 | $C_6H_5-$ | $CH_3-$ | H | OH<br>-CH- | 172-174 |
| 8 | 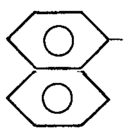 | $CH_3-$ | H | -CO- | 97-99 |
| 9 | 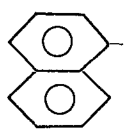 | $CH_3-$ | H | OH<br>-CH- | 174-176 |
| 10 | 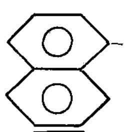 | $CH_3-$ | H | O-$COCH_3$<br>-CH- | 148-150 |
| 11 | 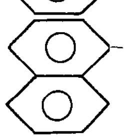 | $CH_3-$ | $C_2H_5-$ | -CO- | 128-129 |
| 12 | 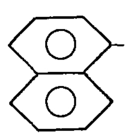 | $CH_3-$ | $C_2H_5$ | OH<br>-CH- | Harz 3105, 3055, 2950, 2845, 1592, 1365, 1212, 1156, 775 |
| 13 | 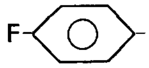 | $CH_3-$ | H | -CO- | 88-90 |

| Beispiel Nr. | Ar | $R^1$ | $R^2$ | X | Fp/°C IR (Film) $[cm^{-1}]$ |
|---|---|---|---|---|---|
| 14 | 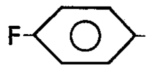 | $CH_3-$ | H | $-\overset{OH}{\overset{\vert}{C}}H-$ | 140-142 |
| 15 | 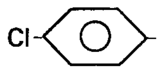 | $CH_3-$ | $C_2H_5-$ | $-CO-$ | Harz 3095, 2950, 2835, 1695, 1472, 1260, 1147, 1125, 1080, 805, 670 |
| 16 | 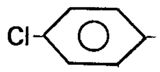 | $CH_3-$ | $C_2H_5-$ | $-\overset{OH}{\overset{\vert}{C}}H-$ | 144-154 |
| 17 | 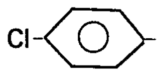 | $CH_3-$ | $n\text{-}C_3H_7-$ | $-CO-$ | Harz 3110, 2960, 2870, 1712, 1490, 1274, 1100, 1022, 812, 705, 678 |
| 18 | 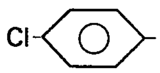 | $CH_3-$ | $n\text{-}C_3H_7-$ | $-\overset{OH}{\overset{\vert}{C}}H-$ Diastereomer I | 125-130 |
| 19 | 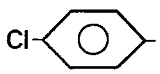 | $CH_3-$ | $n\text{-}C_3H_7-$ | $-\overset{OH}{\overset{\vert}{C}}H-$ Diastereo-merengemisch | Harz 3200, 2960, 2860, 1505, 1490, 1268, 1158, 1130, 1095, 1020, 930, 825, 810 |
| 20 | 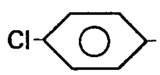 | $CH_3-$ | $-CH(CH_3)_2$ | $-\overset{OH}{\overset{\vert}{C}}H-$ Diastereomer I | 135-155 |
| 21 | 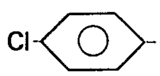 | $CH_3-$ | $-CH(CH_3)_2$ | $-\overset{OH}{\overset{\vert}{C}}H-$ Diastereo-merengemisch | Harz 3270, 2960, 2870, 1886, 1390, 12. ., 1130, 1090, 804, 950, 672 |
| 22 | 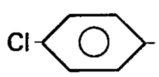 | $CH_3-$ | $n\text{-}C_4H_9$ | $-\overset{OH}{\overset{\vert}{C}}H-$ Diastereomer I | 123-133 |
| 23 | 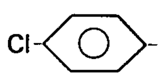 | $CH_3-$ | $n\text{-}C_4H_9$ | $-\overset{OH}{\overset{\vert}{C}}H-$ Diastereo-merengemisch | Harz 2260, 3095, 2955, 2850, 1505, 140. ., 1274, 1102, 1082, 812 |
| 24 | 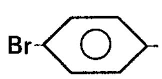 | $CH_3-$ | H | $-CO-$ | Harz 3105, 2965, 2842, 1705, 1470, 13. ., 1253, 1150, 1080, 775, 670 |
| 25 | 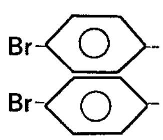 | $CH_3-$ | H | $-\overset{OH}{\overset{\vert}{C}}H-$ | 156-158 |
| 26 | | $CH_3-$ | $C_2H_5$ | $-CO-$ | Harz 3102, 2960, 2920, 2845, 1705, 1475, 1390, 1260, 1150, 1080, 800, 668 |
| 27 | 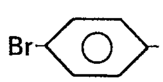 | $CH_3-$ | $C_2H_5-$ | $-\overset{OH}{\overset{\vert}{C}}H-$ Diastereomer I | 172-173 |

| Beispiel Nr. | Ar | $R^1$ | $R^2$ | X | Fp/°C IR (Film) [$cm^{-1}$] |
|---|---|---|---|---|---|
| 28 | 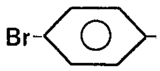 | $CH_3-$ | $C_2H_5-$ | OH<br>$-CH-$<br>Diastereo-merengemisch | Harz 3260, 3020, 2960, 2850, 1495, 1385, 1260, 1125, 1080, 775, 670 |
| 29 | 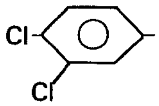 | $CH_3-$ | $C_2H_5$ | $-CO-$ | Harz 3106, 2965, 2850, 1705, 1460, 1385, 1265, 1125, 1024, 918, 810, 750 |
| 30 | 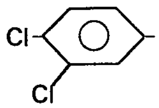 | $CH_3-$ | $C_2H_5-$ | OH<br>$-CH-$<br>Diastereomer I | 171-173 |
| 31 | 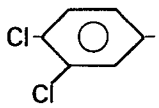 | $CH_3-$ | $C_2H_5-$ | OH<br>$-CH-$<br>Diastereo-merengemisch | Harz 3200, 2985, 2840, 1460, 1384, 126., 1120, 1020, 910, 815, 692 |
| 32 | 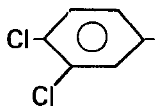 | $CH_3-$ | H | $-CO-$ | 118-120 |
| 33 | 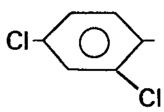 | $CH_3-$ | H | OH<br>$-CH-$ | 133-136 |
| 34 | 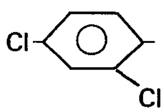 | $CH_3-$ | $C_2H_5-$ | $-CO-$ | Harz 3100, 2955, 2840, 1698, 1570, 1458, 1260, 1150, 1125, 1090, 920, 825 |
| 35 | 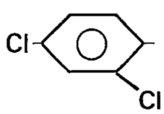 | $CH_3-$ | $C_2H_5-$ | OH<br>$-CH-$ | 138-143 |
| 36 | 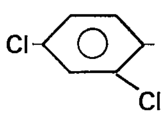 | $CH_3-$ | $n-C_3H_7-$ | $-CO-$ | Harz 3100, 2950, 2860, 1705, 1580, 1466, 1380, 1268, 1096, 825, 672 |
| 37 | 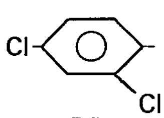 | $CH_3-$ | $n-C_3H_7$ | OH<br>$-CH-$ | 127-129 |
| 38 | 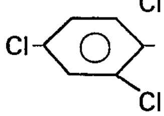 | $CH_3-$ | $-CH(CH_3)_2$ | $-CO-$ | Harz 3105, 2950, 2860, 1705, 1578, 1460, 1380, 1260, 1155, 1095, 922, 825, 670 |
| 39 | 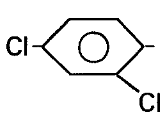 | $CH_3-$ | $-CH(CH_3)_2$ | OH<br>$-CH-$<br>Diastereomer I | 141-143 |

| Beispiel Nr. | Ar | $R^1$ | $R^2$ | X | Fp/°C IR (Film) [$cm^{-1}$] |
|---|---|---|---|---|---|
| 40 | 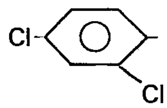 | $CH_3-$ | $-CH(CH_3)_2$ | OH<br>$-CH-$<br>Diastereo-merengemisch | Harz 3130, 2955, 2855, 1735, 1466, 1360, 1228, 1130, 1098, 940, 910, 860 |
| 41 | 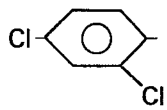 | $CH_3-$ | $-CH(CH_3)_2$ | $O-COCH_3$<br>$-CH-$ | 118-128 |
| 42 | 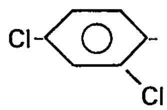 | $CH_3-$ | $n-C_4H_9$ | $-CO-$ | Harz 3100, 2950, 2855, 1706, 1580, 1465, 1264, 1150, 1095, 920, 824, 668 |
| 43 | 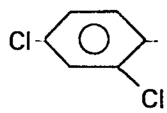 | $CH_3$ | $n-C_4H_9$ | OH<br>$-CH-$ | 153-165 |
| 44 | 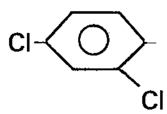 | $CH_3-$ | $n-C_4H_9$ | $O-COCH_3$<br>$-CH-$ | Harz 3110, 2950, 2850, 1740, 1467, 1220, 1130, 1100, 1025, 948, 905, 812, 67 |
| 45 | 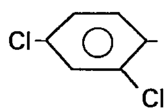 | $CH_3-$ | $-C(CH_3)_3$ | $-CO-$ | 165-168 |
| 46 | 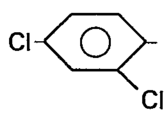 | $CH_3-$ | $-C(CH_3)_3$ | OH<br>$-CH-$ | Harz 3320, 2960, 2860, 1465, 1267, 1188, 1128, 1095, 860, 820, 692 |
| 47 | 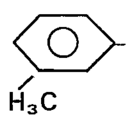 | $CH_3$ | H | $-CO-$ | 53-55 |
| 48 | 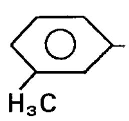 | $CH_3-$ | H | OH<br>$-CH-$ | 128-130 |
| 49 | 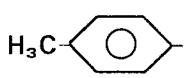 | $CH_3-$ | H | $-CO-$ | 112-114 |
| 50 | 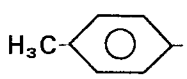 | $CH_3-$ | H | OH<br>$-CH-$ | 114-116 |
| 51 | 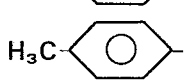 | $CH_3-$ | $C_2H_5$ | $-CO-$ | Harz 3110, 2965, 2850, 1706, 1490, 1380, 1265, 1155, 1082, 915, 800, 630 |
| 52 | 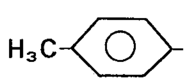 | $CH_3-$ | $C_2H_5-$ | OH<br>$-CH-$<br>Diastereomer I | 161-162 |

| Beispiel Nr. | Ar | $R^1$ | $R^2$ | X | Fp/°C IR (Film) [$cm^{-1}$] |
|---|---|---|---|---|---|
| 53 | 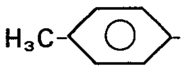 | $CH_3-$ | $C_2H_5-$ | OH<br>$-CH-$<br>Diastereo-merengemisch | Harz 3280, 2965, 2845, 1500, 1385, 1262, 1190, 1080, 918, 870, 800, 670 |
| 54 | 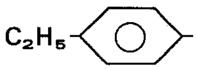 | $CH_3-$ | H | $-CO-$ | 59-60 |
| 55 | 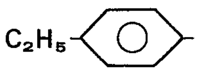 | $CH_3-$ | H | OH<br>$-CH-$ | 111-114 |
| 56 | 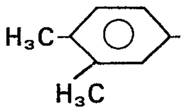 | $CH_3-$ | H | $-CO-$ | Harz 3118, 2970, 2855, 1710, 1492, 1267, 1155, 1078, 920, 807, 672 |
| 57 | 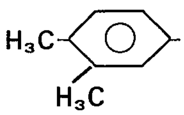 | $CH_3-$ | H | OH<br>$-CH-$ | 127-128 |
| 58 | 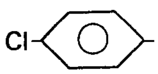 | $CH_3-$ | $-CH(CH_3)_2$ | $-CO-$ | Harz 3100, 2960, 2840, 1710, 1270, 1120, 1080, 1025, 810, 705, 678 |
| 59 | 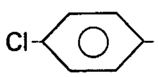 | $CH_3-$ | $n-C_4H_9-$ | $-CO-$ | Harz 3105, 2950, 2845, 1705, 1260, 1150, 1125, 1080, 1022, 808, 670 |
| 60 | 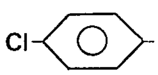 | $C_2H_5-$ | $C_2H_5-$ | $-CO-$ | 89-91 |
| 61 | 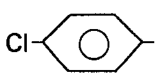 | $C_2H_5-$ | $C_2H_5-$ | OH<br>$-CH-$ | 115-117 |
| 62 | 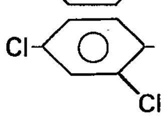 | $C_2H_5$ | $C_2H_5-$ | $-CO-$ | 61-63 |
| 63 | 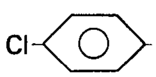 | $C_2H_5-$ | $C_2H_5$ | OH<br>$-CH-$ | 113-115 |

Die neuen Verbindungen können praktisch alle Entwicklungsstadien einer Pflanze verschiedenartig beeinflussen und werden deshalb als Wachstumsregulatoren eingesetzt.

Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation),

d) von den geoklimatischen Faktoren, z.B. Sonnenschein, Dauer, Durchschnittstemperatur, Niederschlagsmenge,

e) von der Bodenbeschaffenheit (einschliesslich Düngung),

f) von der Formulierung bzw. Anwendungsform des Wirkstoffs, und

g) von den angewendeten Konzentrationen der aktiven Substanz.

Aus der Reihe der verschiedenartigen Anwendungsmöglichkeiten der erfindungsgemässen Pflanzenwachstumsregulatoren im Pflanzenanbau, in der Landwirtschaft und im Gartenbau, werden einige nachstehend erwähnt.

Mit den erfindungsgemäss verwendbaren Verbindungen lässt sich das vegetative Wachstum der Pflanzen stark hemmen, was sich insbesondere in einer Reduzierung des Längenwachstums äussert. Die behandelten Pflanzen weisen demgemäss einen gedrungenen Wuchs auf; ausserdem ist eine dunklere Blattfärbung zu beobachten.

Als vorteilhaft für die Praxis erweist sich z.B. die Verringerung des Grasbewuchses an Strassenrändern, Kanalböschungen und auf Rasenflächen wie Park-, Sport- und Obstanlagen, Zierrasen und Flugplätzen, so dass der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des Lagerns (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumvolle. Damit wird ein vollständig mechanisiertes Beernten dieser wichtigen Kulturpflanze ermöglicht.

Durch Anwendung von Wachstumregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumshemmern lässt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide, ist es vorteilhaft, wenn die Bestände durch Behandlung mit erfindungsgemässen Verbindungen im Herbst zwar gut bestockt werden, aber nicht zu üppig in den Winter hineingehen. Dadurch kann der erhöhten Frostempfindlichkeit und – wegen der relativ geringen Blatt- bzw. Pflanzenmasse – dem Befall mit verschiedenen Krankheiten (z.B. Pilzkrankheit) vorgebeugt werden. Die Hemmung des vegetativen Wachstums ermöglicht ausserdem bei vielen Kulturpflanzen eine dichtere Bepflanzung des Bodens, so dass ein Mehrertrag bezogen auf die Bodenfläche erzielt werden kann.

Mit den neuen Wirkstoffen lassen sich Mehrerträge sowohl an Pflanzenteilen als auch an Pflanzeninhaltsstoffen erzielen. So ist es beispielsweise möglich, das Wachstum grösserer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluss zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

Mit Pflanzenwachstumsregulatoren lassen sich schliesslich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, d. h. die Förderung der Ausbildung von Trenngewebe zwischen Frucht- bzw. Blatt und Sprossteil der Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z.B. Getreide wie Weizen, Gerste, Roggen, Hafer und Reis oder Mais oder Gräsern als auch insbesondere bei Dikotylen (z.B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die erfindungsgemässen Wirkstoffe können den Kulturpflanzen sowohl vom Samen her (als Saatgutbeizmittel) als auch über den Boden, d.h. durch die Wurzel sowie – besonders bevorzugt – durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g/kg Saatgut, vorzugsweise 0,01 bis 10 g, benötigt.

Für die Blatt- und Bodenbehandlung sind im allgemeinen Gaben von 0,1 bis 12 kg/ha bevorzugt 0,25 bis 3 kg/ha als ausreichend zu betrachten.

Die erfindungsgemässen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken

des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel oder sonstige oberflächenaktive Mittel wie nichtionogene und anionische Emulgatoren (z.B. Polyoxyethylen/Fettalkohol/Ether, Alkylsulfonate) und Dispergiermittel wie Lignin, Sulfitablaugen und Methylcellulose. Bevorzugt ist die Anwendung der erfindungsgemässen Verbindungen in wässeriger Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gew.-% Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind:

I. 20 Gew.-Teile der Verbindung des Beispiels 7 werden in 3 Gew.-Teilen des Natriumsalzes der Diisobutylnaphthalinsulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

II. 3 Gew.-Teile der Verbindung des Beispiels 29 werden mit 97 Gew.-Teilen feinteiligem Kaolin innig vermischt. Man erhält auf diese Weise ein Stäubemittel, das 3 Gew.-% des Wirkstoffs enthält.

III. 30 Gew.-Teile der Verbindung des Beispiels 35 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 40 Gew.-Teilen der Verbindung des Beispiels 37 werden mit 10 Teilen Natriumsalz eines Phenolsulfonsäureharnstoffformaldehydkondensats, 2 Teilen Kieselgel und 48 Teilen Wasser innig vermischt. Man erhält eine stabile wässerige Dispersion. Durch Verdünnen mit 100000 Gew.-Teilen Wasser erhält man eine wässerige Dispersion, die 0,04 Gew.-% Wirkstoff enthält.

V. 20 Teile der Verbindung des Beispiels 52 werden mit 2 Teilen Calciumsalz der Dodecylbenzolsulfonsäure, 8 Teilen Fettalkoholpolyglykolether, 2 Teilen Natriumsalz eines Phenolsulfonsäureharnstoffformaldehydkondensats und 68 Teilen eines paraffinischen Mineralöls innig vermischt. Man erhält eine stabile ölige Dispersion.

VI. Man vermischt 90 Gew.-Teile der Verbindung des Beispiels 1 mit 10 Gew.-Teilen N-Methylpyrrolidon und erhält eine Lösung, die zur Anwendung in Form kleinster Tropfen geeignet ist.

VII. 20 Gew.-Teile der Verbindung des Beispiels 2 werden in einer Mischung gelöst, die aus 80 Gew.-Teilen Xylol, 10 Gew.-Teilen des Anlagerungsproduktes von 8 bis 10 mol Ethylenoxid an 1 mol Ölsäure-N-monoethanolamid, 5 Gew.-Teilen Calciumsalz der Dodecylbenzolsulfonsäure und 5 Gew.-Teilen des Anlagerungsproduktes von 40 mol Ethylenoxid an 1 mol Rizinusöl besteht. Durch Ausgiessen und feines Verteilen der Lösung in 100000 Gew.-Teilen Wasser erhält man eine wässerige Dispersion, die 0,02 Gew.-% des Wirkstoffs enthält.

VIII. 20 Gew.-Teile der Verbindung des Beispiels 12 werden in einer Mischung gelöst, die aus 40 Gew.-Teilen Cyclohexanon, 30 Gew.-Teilen Isobutanol, 20 Gew.-Teilen des Anlagerungsproduktes von 7 mol Ethylenoxid an 1 mol Isooctylphenol und 10 Gew.-Teilen des Anlagerungsproduktes von 40 mol Ethylenoxid an 1 mol Rizinusöl besteht. Durch Eingiessen und feines Verteilen der Lösung in 100 000 Gew.-Teilen Wasser erhält man eine wässerige Dispersion, die 0,02 Gew.-% des Wirkstoffs enthält.

IX. 20 Gew.-Teile der Verbindung des Beispiels 17 werden in einer Mischung gelöst, die aus 25 Gew.-Teilen Cyclohexanol, 65 Gew.-Teilen einer Mineralölfraktion vom Siedepunkt 210 bis 280° C und 10 Gew.-Teilen des Anlagerungsproduktes von 40 mol Ethylenoxid an 1 mol Rizinusöl besteht. Durch Eingiessen und feines Verteilen der Lösung in 100 000 Gew.-Teilen Wasser erhält man eine wässrige Dispersion, die 0,02 Gew.-% des Wirkstoffs enthält.

Die erfindungsgemässen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrösserung des Wirkungsspektrums. Bei einer Anzahl solcher Wachstumsregulatormischungen treten auch synergistische Effekte auf, d.h. die Wirksamkeit des Kombinationsproduktes ist grösser als die addierten Wirksamkeiten der Einzelkomponenten.

Fungizide, die mit den erfindungsgemässen Verbindungen kombiniert werden können, sind beispielsweise Dithiocarbamate und deren Derivate, wie

Ferridimethyldithiocarbamat,
Zinkdimethyldithiocarbamat,
Manganethylbisthiocarbamat,

Manganzinkethylendiaminbisdithiocarbamat,
Zinkethylenbisthiocarbamat,
Tetramethylthiuramdisulfide,
Ammoniakkomplex von Zink-(N,N-ethylenbisdithiocarbamat), und
N,N'-Polyethylenbis(thiocarbamoyl)disulfid,
Zink-(N,N'-propylenbisdithiocarbamat),
Ammoniakkomplex von Zinn-(N,N'-propylenbis-dithiocarbamat), und
N,N'-Polypropylenbis(thiocarbamoyl)disulfid;

Nitrophenolderivate, wie
Dinitro-(1-methylheptyl)phenylcrotonat,
2-sek.-Butyl-4,6-dinitrophenyl-3,3-dimethylacrylat,
2-sek.-Butyl-4,6-dinitrophenylisopropylcarbonat;

heterocyclische Strukturen, wie
N-Trichlormethylthiotetrahydrophthalimid,
N-Trichlormethylthiophthalimid,
2-Heptadecyl-2-imidazolacetat,
2,4-Dichlor-6-(o-chloranilino)-s-triazin,
O,O-Diethylphthalimidophosphonthionat,
5-Amino-1-[bis(dimethylamino)phosphinyl]-3-phenyl-1,2,4-triazol,
5-Ethoxy-3-trichlormethyl-1,2,4-thiadiazol,
2,3-Dicyano-1,4-dithiaanthrachinon,
2-Thio-1,3-dithio-(4,5-b)-chinoxalin,
1-Butylcarbamoyl-2-benzimidazolcarbaminsäuremethylester,
2-Methoxycarbonylaminobenzimidazol,
2-Rhodanmethylthiobenzthiazol,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-2)-benzimidazol,
Piperazin-1,4-diylbis-1-(2,2,2-trichlorethyl) formamid,
2-Thiazolyl-(4)-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)benzol,
1,2-Bis-(3-methoxycarbonyl-2-thioureido)benzol, und

verschiedene Fungizide, wie
Dodecylguanidinacetat,
3-[2-(3,5-Dimethyl-2-oxycyclohexyl)-2-hydroxyethyl]glutarimid,
Hexachlorbenzol,
N-Dichlorfluormethylthio-N,N'-dimethyl-N-phenylschwefelsäurediamid,
D,L-Methyl-N-(2,6-dimethylphenyl)-N-furyl-(2)-alaninat,
D,L-N-(2,6-Dimethylphenyl)-N-(2'-methoxyacetyl)alaninmethylester,
5-Nitroisophthalsäurediisopropylester,
2,5-Dimethylfuran-3-carbonsäureanilid,
2,5-Dimethylfuran-3-carbonsäurecyclohexylamid,
2-Methylbenzoesäureanilid,
1-(3,4-Dichloranilino)-1-formylamino-2,2,2-trichlorethan,
2,6-Dimethyl-N-tridecylmorpholin bzw. dessen Salze,
2,6-Dimethyl-N-cyclododecylmorpholin bzw. dessen Salze,
2,3-Dichlor-1,4-naphthochinon,
1,4-Dichlor-2,5-dimethoxybenzol,
p-Dimethylaminobenzoldiazinnatriumsulfonat,
1-Chlor-2-nitropropan;

Polychlornitrobenzole, wie Pentachlornitrobenzol, Methylisocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

In dem nachfolgenden Anwendungsbeispiel wird die Wirkung der erfindungsgemäss verwendbaren Stoffe als Pflanzenwachstumsregulatoren dargestellt, ohne die Möglichkeit weiterer Anwendungen als Wachstumsregulatoren auszuschliessen.

*Anwendungsbeispiel:* (Gewächshausversuch)

Zur Bestimmung der wachstumsregulierenden Eigenschaft der Prüfsubstanzen wurden Testpflanzen auf ausreichend mit Nährstoffen versorgtem Torfkultursubstrat in Kunststoffgefässen von ca. 12,5 cm Durchmesser angezogen.

Im Vorlaufverfahren wurden die Testsubstanzen in wässeriger Aufbereitung am Tage der Einsaat auf das Saatbeet gegossen.

Im Nachauflaufverfahren wurden die zu prüfenden Substanzen in wässeriger Aufbereitung auf die Pflanzen gesprüht. Die beobachtete wachstumsregulierende Wirkung wurde bei Versuchsende durch Wuchshöhenmessung belegt. Die so gewonnenen Messwerte wurden zur Wuchshöhe der unbehandelten Pflanzen in Relation gesetzt.

In diesem Versuch, der an Rasen, Tomaten und Soja durchgeführt wurde zeigten insbesondere die Substanzen der Beispiele 1, 2, 7, 13, 16 bis 25, 34, 37, 38, 41 bis 43, 45 und 52 bessere Wirkungen als Chlorcholinchlorid und 3,3-Dimethyl-2-[1,2,4-triazolyl-(1)]-1-(4-chlorbenzoyl)butan.

**Patentansprüche**

1. Verbindungen der Formel (I)

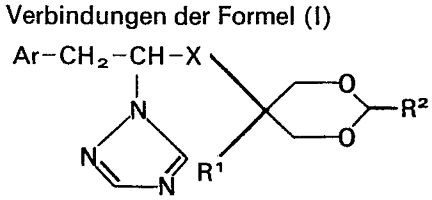

(I)

in der

$R^1$ und $R^2$ verschieden oder gleich sind und Wasserstoff oder Alkyl mit 1 bis 5 C-Atomen bedeuten, Ar Furanyl, Thienyl, Biphenylyl, Naphthyl, oder Phenyl bedeutet, wobei der Phenylrest durch Fluor, Chlor, Brom, Iod, Nitro, Trifluormethyl, weiterhin durch Alkyl, Alkoxy oder Alkenyl mit jeweils 1 bis 5 C-Atomen und ferner durch Phenoxy substituiert sein kann, und

X eine $-CO-$, $-CH(OH)-$ oder $-CH(OR^3)-$-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkynylrest mit 3 bis 4 C-Atomen oder einen $-CO-R^4$-Rest bedeutet, wobei $R^4$ einen Alkylrest mit 1 bis 5 C-Atomen bedeutet.

2. Mittel zur Regulierung des Pflanzenwachstums, enthaltend eine oder mehrere Verbindungen gemäss Anspruch 1.

3. Mittel zur Regulierung des Pflanzenwachstums, enthaltend eine oder mehrere Verbindungen gemäss Anspruch 1 und einen flüssigen oder festen Trägerstoff sowie gegebenenfalls ein oder mehrere oberflächenaktive Mittel.

4. Verwendung von Verbindungen gemäss Anspruch 1 zur Regulierung des Pflanzenwachstums.

5. Verfahren zur Regulierung des Pflanzenwachstums, dadurch gekennzeichnet, dass man eine oder mehrere Verbindungen gemäss Anspruch 1 auf Pflanzen oder deren Lebensraum einwirken lässt.

6. Verfahren zur Herstellung von das Pflanzenwachstum regulierenden Mitteln, dadurch gekennzeichnet, dass man eine oder mehrere Verbindungen gemäss Anspruch 1 mit festen oder flüssigen Trägerstoffen sowie gegebenenfalls einem oder mehreren oberflächenaktiven Mitteln mischt.

7. Verfahren zur Herstellung von 1,3-Dioxan-5-ylalkyltriazolen der Formel (I) gemäss Anspruch 1, dadurch gekennzeichnet, dass man

a) 1,2,4-Triazol mit α-Bromketonen der Formel (II)

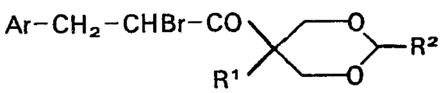 (II)

worin $R^1$, $R^2$ und Ar die oben angegebene Bedeutung haben, oder

b) ein Arylmethylhalogenid der Formel (III)

$$Ar-CH_2-Y \qquad (III)$$

worin Ar die oben genannte Bedeutung hat und Y Chlor oder Brom ist, mit einem 1-(1,3-Dioxan-5-yl)-2-[1,2,4-triazolyl-(s)]-ethan-1-on der Formel (IV)

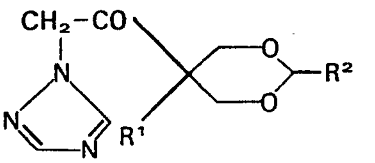 (IV)

worin $R^1$ und $R^2$ die oben genannte Bedeutung haben,

umsetzt und die so erhaltenen Verbindungen gegebenenfalls anschliessend reduziert und – falls gewünscht – danach verethert oder verestert.

## Revendications

1. Composés de formule (I):

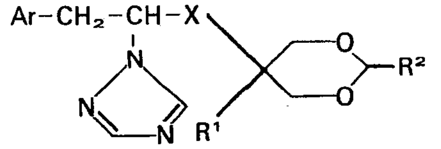 (I)

dans laquelle:

$R^1$ et $R^2$, qui peuvent être identiques ou différents, désignent chacun un atome d'hydrogène ou un radical alkyle en $C_1$ à $C_5$;

Ar représente un groupe furannyle, thiényle, biphénylyle, naphtyle ou phényle, le reste phényle pouvant être substitué par des atomes de fluor, de chlore, de brome ou d'iode ou des groupes nitro, trifluorométhyle, alkyle, alcoxy ou acényle en $C_1$ à $C_5$ et phénoxy, et

X représente un groupe $-CO$, $-CH(OH)-$ ou $-CH(OR^3)-$, où $R^3$ désigne un radical alkyle en $C_1$ à $C_8$, un radical alcényle en $C_2$ à $C_5$ éventuellement chlorosubstitué, un radical alcynyle en $C_3$ ou $C_4$ ou un groupe $-CO-R^4$, où $R^4$ désigne un radical alkyle en $C_1$ à $C_5$.

2. Composition pour la régulation de la croissance de plantes, contenant un ou plusieurs composés selon la revendication 1.

3. Composition pour la régulation de la croissance de plantes, contenant un ou plusieurs composés selon la revendication 1 à côté d'un support solide ou liquide et éventuellement d'un ou de plusieurs agents tensio-actifs.

4. Utilisation de composés selon la revendication 1 pour la régulation de la croissance de plantes.

5. Procédé pour la régulation de la croissance de plantes, caractérisé en ce que l'on fait agir un ou plusieurs composés selon la revendication 1 sur les plantes ou sur leur biotope.

6. Procédé de préparation de compositions pour la régulation de la croissance de plantes, caractérisé en ce que l'on mélange un ou plusieurs composés selon la revendication 1 avec des supports solides ou liquides et éventuellement avec un ou plusieurs agents tensio-actifs.

7. Procédé de préparation de 1,3-dioxanne-5-ylalkyltriazoles de formule (I) selon la revendication 1, caractérisé en ce que l'on fait réagir

soit a) du 1,2,4-triazole avec des α-bromocétones de formule (II):

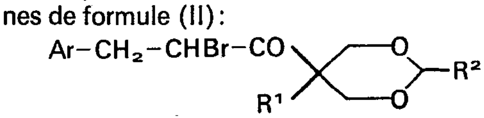 (II)

dans laquelle $R^1$, $R^2$ et Ar possèdent la signification définie,

soit b) un halogénure d'arylméthyle de formule (III):

$$Ar-CH_2-Y \qquad (III)$$

dans laquelle Ar possède la signification définie et Y désigne un atome de chlore ou de brome, avec une 1-(1,3-dioxanne-5-yl)-2-[1,2,4-triazolyl-(s)]-éthane-1-one de formule (IV):

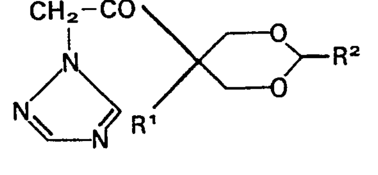 (IV)

dans laquelle $R^1$ et $R^2$ possèdent la signification définie,
les composés obtenus étant, le cas échéant, soumis ensuite à une réduction, suivie éventuellement d'une éthérification ou d'une estérification.

## Claims

1. A compound of the formula (I)

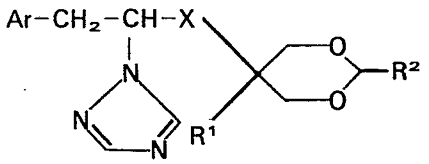

(I)

where $R^1$ and $R^2$ are identical or different and each is hydrogen or alkyl of 1 to 5 carbon atoms, Ar is furanyl, thienyl, biphenylyl or naphthyl, or is phenyl which is unsubstituted or substituted by fluorine, chlorine, bromine, iodine, nitro or trifluoromethyl, or by alkyl, alcoxy or alkenyl, each of 1 to 5 carbon atoms, or by phenoxy, and X is $-CO-$, $-CH(OH)-$ or $-CH(OR^3)-$, where $R^3$ is alkyl of 1 to 8 carbon atoms or unsubstituted or chlorine-substituted alkenyl of 2 to 5 carbon atoms, or alkynyl of 3 or 4 carbon atoms, or $R^3$ is $-CO-R^4$ is alkyl of 1 to 5 carbon atoms.

2. An agent for regulating plant growth, containing one or more compounds as claimed in claim 1.

3. An agent for regulating plant growth, containing one or more compounds as claimed in claim 1 and a solid liquid carrier, together with, if desired, one or more surfactants.

4. The use of a compound as claimed in claim 1 for regulating plant growth.

5. A method of regulating plant growth, wherein one or more compounds as claimed in claim 1 are allowed to act on the plants or their habitat.

6. A process for the production of agents for regulating plant growth, wherein one or more compounds as claimed in claim 1 are mixed with solid or liquid carriers and, if desired, one or more surfactants.

7. A process for manufacturing 1,3-dioxan-5-yl-alkyltriazoles of the formula (I) as claimed in claim 1, wherein

a) a 1,2,4-triazole is reacted with an α-bromoketone of the formula (II)

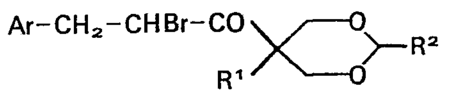

(II)

where $R^1$ and $R^2$ and Ar have the above meanings, or

b) an arylmethyl halide of the formula (III)

$$Ar-CH_2-Y \qquad (III)$$

where Ar as the above meanings and Y is chlorine or bromine, is reacted with a 1-(1,3-dioxan-5-yl)-2-[1,2,4-triazolyl-(s)]-ethan-1-one of the formula (IV)

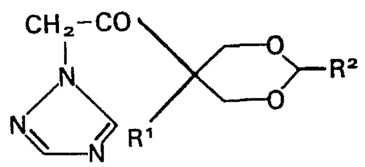

(IV)

where $R^1$ and $R^2$ have the above meanings, and, if desired, the compounds thus obtained are subsequently reduced and then, if desired, etherified or esterified.